(19) Europäisches Patentamt
European Patent Office
Office européen des brevets

(11) Publication number: 0 392 976 A1

(12) **EUROPEAN PATENT APPLICATION**

(21) Application number: 90810260.1

(22) Date of filing: 30.03.90

(51) Int. Cl.5: C12P 21/08, C12N 5/12, A61K 39/395, G01N 33/577

The applicant has filed a statement in accordance with Rule 28 (4) EPC (issue of a sample only to an expert). Accession number(s) of the deposit(s): ECACC 89031404, 89031403, 89031405 and 89032104.

(30) Priority: 08.04.89 GB 8907941

(43) Date of publication of application: 17.10.90 Bulletin 90/42

(84) Designated Contracting States: AT BE CH DE DK ES FR GB GR IT LI LU NL SE

(71) Applicant: CIBA-GEIGY AG
Klybeckstrasse 141
CH-4002 Basel(CH)

(72) Inventor: Rosenfelder, Günter, Dr.
Am Sonnenrain 21
D-7852 Binzen(DE)
Inventor: Towbin, Harry, Dr.
Feldstrasse 31
CH-4123 Allschwil(CH)

(54) **Monoclonal antibodies to tetracyclic compounds, processes for their production, and applications.**

(57) The invention concerns monoclonal antibodies directed against R-(-)-oxaprotiline and its metabolite R-(-)-oxaprotiline beta-glucuronide, derivatives thereof, processes for the preparation of these antibodies and their derivatives, hybridoma cell lines that secrete the desired monoclonal antibodies, a process for the preparation of such hybridoma cells, kits containing the monoclonal antibodies and/or their derivatives, and the use of the monoclonal antibodies and/or their derivatives for the qualitative and quantitative determination of R-(-)-oxaprotiline and R-(-)-oxaprotiline beta-glucuronide and for the treatment of a toxicosis due to an overdose of R-(-)-oxaprotiline.

EP 0 392 976 A1

## Monoclonal antibodies to tetracyclic compounds, processes for their production, and applications

The invention concerns monoclonal antibodies directed against R-(-)-oxaprotiline and its metabolite R-(-)-oxaprotiline beta-glucuronide, derivatives thereof, processes for the preparation of these antibodies and their derivatives, hybridoma cell lines that secrete the desired monoclonal antibodies, a process for the preparation of such hybridomas, test kits containing the monoclonal antibodies and/or their derivatives, and the use of the monoclonal antibodies and/or their derivatives for the qualitative and quantitative determination of R-(-)-oxaprotiline and R-(-)-oxaprotiline beta-glucuronide and for the treatment of a toxicosis due to an overdose of R-(-)-oxaprotiline.

### Background of the invention

R-(-)-oxaprotiline is a tetracyclic compound of the formula

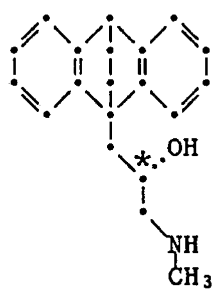

(I).

It is a useful anti-depressant and shows, for example, anti-aggressive and anti-histaminic properties. The production of R-(-)-oxaprotiline is described in the European Patent 0 014 433. In the human organism, R-(-)-oxaprotiline is predominantly metabolized to R-(-)-oxaprotiline beta-glucuronide of the formula

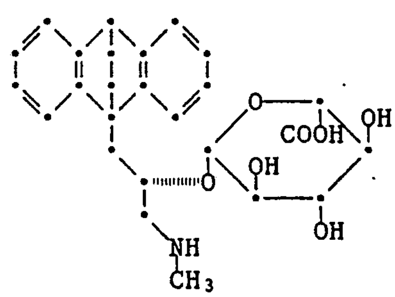

(II)

which is excreted in the urine.

When treating a patient suffering from depression with an antidepressant such as R-(-)-oxaprotiline, it has to be kept in mind that each individual reacts differently owing to the great individual variability of the drug's metabolism in patients and hence of the relation between dose, plasma level and clinical response. It is therefore necessary to ascertain the therapeutically effective dose individually to avoid risks due to overdose and to monitor the antidepressant therapy carefully. Moreover, the success of the therapy relies to a great deal on patient compliance, i.e. the degree in which the doctor's orders with regard to medication are followed. The problem of patient noncompliance is very common which makes it difficult to evaluate the patient's response to the drug and also causes unnecessary costs when prescribed and purchased medication is not taken.

For the above-named purposes, methods to determine the therapeutic compound or its metabolites in whole blood, plasma, serum and/or urine are the most suitable. In principle several analytical techniques exist for measuring the concentration of the drug in body fluids, e.g. gas chromatography, high-pressure liquid chromatography and the like. Few medical services and laboratories can afford to use them for routine purpose due to the high costs and the need for specially trained technicians. Especially in private

doctor's offices it is obviously not feasible to do any drug monitoring that requires sophisticated instrumentation. It is therefore desirable to develop easier and less costly reliable analytical tests for R-(-)-oxaprotiline and/or its metabolites. Most suitable to solve this problem would be immunoassays based on selective monoclonal antibodies directed against R-(-)-oxaprotiline and its metabolites, such as R-(-)-oxaprotiline beta-glucuronide.

## Object of the invention

It is an object of this invention to provide monoclonal antibodies which are highly specific for the antidepressant R-(-)-oxaprotiline and its metabolite R-(-)-oxaprotiline beta-glucuronide. The monoclonal antibodies can be used to monitor the distribution and concentration of the drug in whole blood, plasma or serum and to detect and determine the metabolite in the urine. Therefore, they are valuable tools in monitoring an antidepressant therapy with R-(-)-oxaprotiline with regard to effective dose determination and patient compliance. Moreover, such monoclonal antibodies may be used to detoxify overdosed patients due to their high binding affinity to R-(-)-oxaprotiline.

## Description of the invention

The invention concerns monoclonal antibodies directed against R-(-)-oxaprotiline and/or its major metabolite R-(-)-oxaprotiline beta-glucuronide, preferentially directed against R-(-)-oxaprotiline, and derivatives thereof which retain their specificity for the antigenic determinants of said compounds.

Preferred are monoclonal antibodies and derivatives thereof which show little or no crossreactivity with substances which are structurally similar to R-(-)-oxaprotiline and its beta-glucuronide, especially monoclonal antibodies and derivatives thereof which selectively bind to R-(-)-oxaprotiline in the presence of other dibenzobicyclo[2.2.2]octadiene compounds which differ from R-(-)oxaprotiline by chemical modifications of the tetracyclic ring structure and/or the aliphatic side chain. Also preferred are monoclonal antibodies and derivatives thereof which selectively bind to R-(-)-oxaprotiline in the presence of other therapeutic drugs lacking the dibenzobicyclo[2.2.2]octadiene skeleton, which are potentially used in combination with R-(-)-oxaprotiline, for example neuroleptics, anxiolytics, psychotropics, anti-epileptics or anti-arrhythmics. Also preferred are monoclonal antibodies and derivatives thereof which selectively bind to R-(-)-oxaprotiline in the presence of S-(+)-oxaprotiline.

Most preferred are the monoclonal antibodies with the designation MAb 1219S59.11, MAb 1219S78.6, MAb 1220S36.3, MAb 1225S93.2, MAb 1226S31.3, MAb 1228P54.6 and MAb 1228P63.1, especially MAb 1219S59.11, MAb 1219S78.6, MAb 1226S31.3 and MAb 1228P63, and derivatives thereof.

The affinities of the monoclonal antibodies of the invention to R-(-)-oxaprotiline can for example be determined in an inhibition enzyme immunoassay according to Friguet et al. (J. Immunol. Methods 77, 305, 1985) wherein the monoclonal antibody to be tested is preincubated with R-(-)-oxaprotiline and, in a second step, exposed to a solid phase coated with an adsorbable conjugate of R-(-)-oxaprotiline, e.g. a protein conjugate. The fraction of the monoclonal antibody not engaged in immunocomplexes binds to the solid phase and is quantified by a secondary antibody-enzyme conjugate directed against the monoclonal antibody. The affinities are expressed as $IC_{50}$ (inhibition concentration) values, i.e. the concentration of free antigen causing a 50 % inhibition of antibody binding to the solid phase antigen.

The selectivity of the monoclonal antibodies of the invention is tested by analyzing inhibition of monoclonal antibody binding to R-(-)-oxaprotiline by structurally similar compounds, e.g. by other dibenzobicyclo[2.2.2]octadiene compounds which differ from R-(-)-oxaprotiline by chemical modifications of the tetracyclic ring structure and/or the aliphatic side chain, by therapeutic compounds lacking the dibenzobicyclo[2.2.2]octadiene skeleton which are potentially used in combination with the antidepressant, and by the enantiomer S-(+)-oxaprotiline. This can be done, for example, in an inhibition enzyme immunoassay wherein the monoclonal antibodies of the invention are preincubated with any of the soluble compounds listed above and the incubation mixture is then tested in the enzyme immunoassay described above.

Derivatives of monoclonal antibodies of this invention retain their specificity for the antigenic determinants of R-(-)-oxaprotiline and/or R-(-)-oxaprotiline beta-glucuronide, i.e. they retain the characteristic binding pattern of the parent antibody to said compounds. Examples of such derivatives are conjugates of the monoclonal antibodies with an enzyme, a fluorescent marker, a chemiluminescent marker, a metal chelate, avidin, biotin or the like, radioactively labelled antibodies or antibody fragments.

Enzymes used for antibody conjugates of the invention are, for example, horseradish peroxidase, alkaline phosphatase, β-D-galactosidase, glucose-oxidase, glucoamylase, carboanhydrase, acetylcholinesterase, lysozyme, malate dehydrogenase or glucose-6-phosphate dehydrogenase. Fluorescent markers conjugated with the monoclonal antibodies of the invention are fluorescein, fluorochrome, rhodamine and the like. Chemiluminescent markers are e.g. acridinium esters or luminol. In such conjugates the antibody is bound to the conjugation partner directly or by the way of a spacer or linker group. Examples for metal chelators are ethylenediaminetetraacetic acid (EDTA), diethylenetriaminepentaacetic acid (DPTA), 1,4,8,11-tetraazatetradecane, 1,4,8,11-tetraazatetradecane-1,4,8,11-tetraacetic acid, 1-oxa-4,7,12,15-tetraazaheptadecane-4,7,12,15-tetraacetic acid, or the like.

Radioactively labelled monoclonal antibodies contain e.g. radioactive iodine ($^{123}I$, $^{125}I$, $^{131}I$), yttrium ($^{90}Y$), technetium ($^{99m}Tc$),or the like.

Antibody fragments of the invention are for example the univalent fragments Fab (Fab = fragment antigen binding) or Fab′ and the divalent fragment F(ab′)2.

The monoclonal antibodies of the invention and derivatives thereof are obtained by processes known per se, characterized in that hybridoma cells as defined below secreting the desired monoclonal antibodies are multiplied according to known methods in vitro or in vivo. When required, the resulting monoclonal antibodies are isolated and/or converted into derivatives thereof.

Multiplication in vitro is carried out in suitable culture media, which are the customary standard culture media, for example Dulbecco's Modified Eagle Medium (DMEM) or RPMI 1640 medium, optionally replenished by a mammalian serum, e.g. fetal calf serum, or trace elements and growth-sustaining supplements, e.g. feeder cells such as normal mouse peritoneal exudate cells, spleen cells or bone marrow macrophages, 2-aminoethanol, insulin, transferrin, low density lipoprotein, oleic acid, or the like.

In vitro production provides relatively pure antibody preparations and allows scale-up to give large amounts of the desired antibodies. Techniques for large scale hybridoma cultivation under tissue culture conditions are known in the art and include homogeneous suspension culture, e.g. in an airlift reactor or in a continuous stirrer reactor, or immobilized or entrapped cell culture, e.g. in hollow fibres, micro-capsules, on agarose microbeads or ceramic cartridges.

For isolation of the monoclonal antibodies, the immunoglobulins in the culture supernatants are first concentrated e.g. by precipitation with ammonium sulphate, dialysis against hygroscopic material such as polyethylene glycol (PEG), filtration through selective membranes or the like. If necessary and/or desired, the concentrated antibodies are purified by customary chromatography methods, for instance gel filtration, ion exchange chromatography, chromatography over DEAE-cellulose or Protein A, or immunoaffinity chromatography.

Large amounts of the desired monoclonal antibodies can also be obtained by multiplying hybridoma cells in vivo. Cell clones are injected into mammals which are histocompatible with the parent cells, e.g. syngeneic mice, to cause growth of antibody-producing tumors. optionally, the animals are primed with a hydrocarbon, especially mineral oils such as pristane (tetramethylpentadecane), prior to the injection. As an example, hybridoma cells derived from Balb/c mice are intraperitoneally injected into Balb/c mice optionally pre-treated with pristane, and after one to two weeks ascites fluid of these mice is collected. The desired monoclonal antibodies are isolated from the body fluids by conventional methods as described above.

Conjugates of antibodies of the invention are prepared by methods known in the art, e.g. by reacting a monoclonal antibody prepared as described hereinbefore with an enzyme in the presence of a coupling agent, e.g. glutaraldehyde, periodate, N,N′-o-phenylenedimaleimide, N-(m-maleimidobenzoyloxy)-succinimide, N-(3-[2′-pyridyldithio]-propionoxy)-succinimide, N-ethyl-N′-(3-dimethylaminopropyl)-carbodiimide or the like. Conjugates with avidin are prepared likewise. Conjugates with biotin are prepared e.g. by reacting monoclonal antibodies with an activated ester of biotin such as the biotin N-hydroxysuccinimide ester. Conjugates with fluorescent or chemiluminescent markers are prepared in the presence of a coupling agent, e.g. those listed above, or by reaction with an isothiocyanate, preferably fluorescein-isothiocyanate. Antibody conjugates with metal chelates are prepared in an analogous manner.

Monoclonal antibodies radioactively labelled with iodine ($^{123}I$, $^{125}I$, $^{131}I$) are obtained from the monoclonal antibodies according to the invention by iodination known per se, for example with radioactive sodium or potassium iodide and a chemical oxidising agent, such as sodium hypochlorite, chloramine T or the like, or an enzymatic oxidising agent, such as lactoperoxidase, or glucose oxidase and glucose. Monoclonal antibodies according to the invention are coupled to yttrium ($^{90}Y$) for example by diethylenetriaminepentaacetic acid (DPTA) chelation. Technetium-99m labelled antibodies are prepared by ligand exchange processes, for example by reducing pertechnate ($TcO_4^-$) with stannous ion solution, chelating the reduced technetium onto a sephadex column and applying the antibody to this column, or by direct labelling techniques, e.g. by incubating pertechnate, a reducing agent such as $SnCl_2$, a buffer solution such as a sodium potassium

phthalate solution, and the antibody.

Fragments of monoclonal antibodies, for example Fab, Fab′ or $F(ab')_2$ fragments, can be obtained from the antibodies prepared as described above by methods known per se, e.g. by digestion with enzymes such as pepsin or papain and/or cleavage of disulfide bonds by chemical reduction.

The invention further concerns hybridoma cell lines, characterized in that they secrete monoclonal antibodies as described hereinbefore directed against R-(-)-oxaprotiline and/or R-(-)-oxaprotiline beta-glucuronide, especially directed against R-(-)-oxaprotiline.

In particular the invention concerns cell lines which are hybrids of myeloma cells, especially mouse myeloma cells, and B lymphocytes of a mammal, preferentially a syngeneic mouse, immunized with an immunogenic R-(-)-oxaprotiline conjugate. Suitable immunogenic conjugates of R-(-)-oxaprotiline are described below.

Preferred are the hybridoma cell lines with the designation 1219S59.11, 1219S78.6, 1220S36.6, 1225S93.2, 1226S31.3, 1228P54.6 and 1228P63.1. The hybridoma cell lines with the designation 1219S59.11, 1219S78.6, 1220S36.6, 1225S93.2 and 1226S31.3 are hybrids of the mouse myeloma cell line Sp2/O-Ag14 and of B lymphocytes of the spleen of Balb/c mice immunized with conjugates of R-(-)-oxaprotiline with bovine serum albumin (BSA). The hybridoma cell lines with the designation 1228P54.6 and 1228P63.1 are hybrids of the mouse myeloma cell line PAI and B lymphocytes of the spleen of Balb/c mice immunized with conjugates of R-(-)-oxaprotiline with BSA. Particularly preferred are the hybridoma cell lines 1219S59.11, 1219S78.6, and 1228P63.1, which have been deposited on March 14, 1989, at the European Collection of Animal Cell Cultures (E.C.A.C.C.), PHLS Centre for Applied Microbiology & Research, Porton Down, Salisbury, Wilts. SP4 OJG, U.K., under the deposition numbers 89031404, 89031403, and 89031405, respectively, and the hybridoma cell line 1226S31.3 which has been deposited at the E.C.A.C.C. on March 21, 1989, under the deposition number 89032104.

The hybridoma cell lines of the invention are genetically stable, secrete the monoclonal antibodies of the invention with constant specificity and may be kept in deep-frozen cultures and reactivated by thawing and optional re-cloning.

The invention concerns also a process for the production of hybridoma cell lines secreting the monoclonal antibodies of the invention, characterized in that a suitable mammal is immunized with an immunogenic R-(-)-oxaprotiline conjugate, antibody producing cells of this mammal are fused with cells of a continuous cell line, the hybrid cells obtained in the fusion are cloned, and cell clones secreting the desired antibodies are selected.

In order to obtain high yields of antibodies, it is necessary to use an immunogenic conjugate of R-(-)-oxaprotiline for the immunization, since R-(-)-oxaprotiline is a hapten and not immunogenic by itself. Suitable R-(-)-oxaprotiline conjugates are described in the following. In the conjugate, R-(-)-oxaprotiline is coupled to a carrier molecule. Suitable carrier molecules are for example high molecular weight proteins like bovine serum albumin (BSA; MW 66,200), alpha-amylase from Bacillus subtilis (MW 58,000) or keyhole limpet hemocyanin (KLK; MW > 1,000,000) which are commercially available in large quantities. Porcine thyroglobulin, toxins such as tetanus, cholera or diphteria toxins, human serum albumin (HSA), beta-2 microglobulin, and the like, and purified rabbit IgG fraction against mouse IgG(H+L) (Kawamura & Berzofsky, J. Immunol. 136, 58, 1986) may also be employed as carriers. Other possible carrier molecules include polysaccharides, natural or synthetic lipopolysaccharides, synthetic polypeptides such as polylysine, activated membranes, latex particles, bacteria such as Salmonella, and the like. Preferred is an immunogenic R-(-)-oxaprotiline conjugate in which R-(-)-oxaprotiline is coupled to bovine serum albumin (BSA).

The R-(-)-oxaprotiline conjugates of the invention are prepared by methods known per se, either by adsorption of R-(-)-oxaprotiline to the carrier or by coupling using carbodiimides, periodate, glutaraldehyde, N,N′-o-phenylenedimaleimide, N-(m-maleimidobenzoyloxy)-succinimide, N-(3-[2′-pyridyldithio]-propionoxy)-succinimide or the like.

The immunogenic R-(-)-oxaprotiline conjugate may be mixed with adjuvants, i.e. agents that will further increase the immune response, for the immunization procedure. Possible adjuvants are Freund's complete adjuvant (emulsion of mineral oil, water, and mycobacterial extracts), Freund's incomplete adjuvant (emulsion of water and oil only), aluminium hydroxide gels etc.

The immunogenic R-(-)-oxaprotiline conjugate is used to immunize suitable mammals which recognize the conjugate as a foreign molecule, especially mice or rats, preferentially mice. Particularly preferred are Balb/c mice.

The routes of immunization include, among others, intradermal, subcutaneous, intramuscular, intraperitoneal, intravascular and intracranial injections. Since high antibody titers are desired, a series of injections is commonly given. The immunization is for example performed by injecting the immunogenic R-(-)-oxaprotiline conjugate, optionally mixed with incomplete or complete Freund's adjuvant, three to eight times parenterally, e.g. intraperitoneally and/or subcutaneously, in amounts of around 30 µg into Balb/c mice at intervals of 1-3 weeks, followed by a booster injection of about 30 µg 1-3 days prior to sacrificing the animals.

Antibody-producing cells of the immunized mammals, preferably lymphoid cells such as spleen lymphocytes, taken for example 1-3 days after the final booster injection, are fused with the cells of a continuous cell line, i.e. a continuously replicating cell clone which confers this replication ability to the hybrid cells resulting from the fusion. An example for such a cell line is a tumor cell line (myeloma) which does not itself produce immunoglobulins or fragments thereof but has the potential to produce and secrete large amounts of antibody, and which carries a genetic marker so that the hybrid cells can be selected against non-fused parent cells. Several suitable myeloma cell lines are known in the art. Preferred are myeloma cell lines lacking the enzyme hypoxanthine guanine phosphoribosyl transferase (HGPRT) or the enzyme thymidine kinase (TK), which therefore do not survive in a selective culture medium containing hypoxanthine, aminopterin and thymidine (HAT medium). Particularly preferred are myeloma cells and derived cell lines that do not survive in HAT medium and do not secrete immunoglobulins or fragments thereof, such as the cell lines Sp2/O-Ag14 or PAI.

The fusion is performed in the presence of a fusion promoter, for example Sendai virus or other paramyxo viruses, optionally in UV-inactivated form, of chemical fusogens such as calcium ions, surface-active lipids, e.g. lysolecithin, or polyethylene glycol (PEG), or by electrofusion. Preferentially, the myeloma cells are fused with a three- to twentyfold excess of spleen cells from immunized mammals in a solution containing about 30 % to about 60 % polyethylene glycol of a molecular weight between 1000 and 4000.

After the fusion, the cells are resuspended and cultivated in a selective medium, for example HAT medium. In this medium, only hybridoma cells will survive, because they combine the ability to grow and replicate in vitro like the parent myeloma cells and the missing HGPRT or TK genes essential for the survival in the HAT medium inherited from the antibody-producing spleen cells of the immunized mammals.

Suitable culture media for the expansion of hybridoma cells are the standard culture media, such as Dulbecco's modified Eagle medium (DMEM), minimum essential medium, RPMI 1640 medium and the like, optionally replenished by a mammalian serum, e.g. 10 to 15 % fetal calf serum. Preferentially, feeder cells, e.g. normal mouse peritoneal exudate cells, spleen cells, bone marrow macrophages, or the like, are added at the beginning of the cell growth immediately after the fusion step to nourish the hybridoma cells and support their growth, especially where cell densities are low, by providing growth factors and the like. If phago cytic cells such as macrophages or monocytes are used, they can perform a helpful service in cleaning up the debris of dead myeloma cells always found after aminopterin treatment. The culture media are supplemented with selective medium in order to prevent myeloma cells from overgrowing the hybridoma cells.

The hybridoma cell culture supernatants are screened for the desired monoclonal antibodies, preferentially with an enzyme immunoassay or a radioimmunoassay. Positive hybridoma cells are cloned, e.g. by limiting dilution or in soft agar, preferentially twice or more. Optionally, hybridoma cells are passaged through animals, e.g. mice, by intraperitoneal injection and harvesting of ascites, which stabilizes hybridomas and improves growth characteristics. The cloned cell lines may be frozen in a conventional manner.

The monoclonal antibodies of the invention and/or their derivatives are useful for the qualitative and quantitative determination of R-(-)-oxaprotiline or R-(-)-oxaprotiline beta-glucuronide, for example in whole blood, plasma or serum for determination of the effective dosis and therapeutic drug monitoring or in urine for patient compliance testing.

For instance, the antibodies or derivatives thereof can be used in any of the known immunoassays which rely on the binding interaction between the antigenic determinants of R-(-)-oxaprotiline and R-(-)-oxaprotiline beta-glucuronide, respectively, and the paratopes of the monoclonal antibodies. Such immunoassays are less costly and time consuming than known methods and lead to reliable and reproducible results without the requirement for complicated and expensive instrumentation. Examples of suitable immunoassays assays are radioimmunoassays (RIA), enzyme, immuno-fluorescence, chemiluminescence, immunoprecipitation, latex agglutination, or hemagglutination immunoassays. Such immunoassays are useful e.g. in the qualitative and quantitative determination of the drug or its metabolite in biological fluids, e.g. in whole blood, plasma, serum and urine, respectively, of patients under therapy with the antidepressant.

The antibodies according to the invention can be used as such or in the form of radioactively labelled derivatives in a radioimmunoassay (RIA). Any of the known modifications of a RIA suitable for small molecules can be used, for example soluble phase (homogeneous) RIA, solid phase (heterogeneous) RIA,

single RIA or double (sandwich) RIA with direct or indirect (competitive) determination of R-(-)-oxaprotiline or R-(-)-oxaprotiline beta-glucuronide.

An example of such a radioimmunoassay is a RIA in which a solution containing an unknown amount of R-(-)-oxaprotiline or R-(-)-oxaprotiline beta-glucuronide is incubated with a monoclonal antibody of the invention and radiolabelled, e.g. $^{123}$I-, $^{125}$I-, $^{131}$I-, $^{14}$C- or $^{3}$H-labelled, R-(-)-oxaprotiline, the immune complex formed is separated and the amount of radioactivity in the immune complex measured. The separation is carried out by precipitation of the immune complex by the addition of irrelevant immunoglobulin, e.g. bovine, equine, rabbit or rat immunoglobulin, followed by addition of suitable solvents, e.g. polyethylene glycol. Otherwise the immune complex may be adsorbed on affinity chromatography material with high affinity for the monoclonal antibodies of the invention, e.g. chromatography material coupled to protein A or to an antibody recognizing and binding the monoclonal antibodies of the invention, such as to rabbit anti-mouse immunoglobulin. To facilitate separation of the immune complex, the monoclonal antibody of the invention may be replaced by a conjugate of such a monoclonal antibody with a solid carrier, for example a bead, a filterable particle or a paramagnetic particle which can be collected and separated with the aid of a magnet. The carrier material may be of inorganic or organic origin, e.g. a plastic or glass bead, filterable particles useful as carriers in immunoaffinity chromatography such as crosslinked agarose, crosslinked dextran or polyacrylamide particles, or inorganic paramagnetic oxide particles.

The monoclonal antibodies according to the invention can be used as such or in the form of enzyme-conjugated derivatives in an enzyme immunoassay. As described above for radioimmunoassays, any of the known modifications of an enzyme immunoassay suitable for small molecules can be used.

The tests are carried out in an analogous manner to the radioimmunoassays described above using an enzyme label instead of a radioactive label. The amount of immune complex formed is determined by adding an enzyme substrate solution. The enzyme substrate reaction results, for example, in a colour change which can be observed by eye or with optical measuring devices.

The monoclonal antibodies according to the invention can be used as such or in the form of derivatives of the invention conjugated with chemiluminescent markers in chemiluminescence assays. As described above for radioimmunoassays, any of the known modifications of a chemiluminescence assay suitable for small molecules can be used.

The tests are carried out in an analogous manner to the radioimmunoassays described above using a chemiluminescent label, e.g. acridinium ester, instead of a radioactive label. The amount of immune complex formed which corresponds to the amount of antigen present in the test solution is determined by adding a compound triggering luminescence, e.g. $H_2O_2$ and NaOH, and measuring the emission of light with optical measuring devices.

The use according to the invention of monoclonal antibodies and derivatives thereof as described hereinbefore for the qualitative and quantitative determination of R-(-)-oxaprotiline or R-(-)-oxaprotiline beta-glucuronide also includes other immunoassays suitable for small molecules known per se, for example immunofluorescence assays, latex agglutination with antibody-coated or antigen-coated latex particles, hemagglutination with antibody-coated or antigen-coated red blood corpuscles, evanescent light wave assays using an antibody-coated optical fibre and other direct-acting immunosensors which convert the binding event into an electrical or optical signal, or the like.

The invention relates also to test kits for the qualitative and quantitative determination of R-(-)-oxaprotiline or R-(-)-oxaprotiline beta-glucuronide comprising monoclonal antibodies of the invention and/or derivatives thereof and, optionally, other monoclonal or polyclonal antibodies and/or adjuncts.

Test kits according to the invention for a radioimmunoassay comprise, for example, a suitable carrier, e.g. microtitre plates or paramagnetic particles, uncoated or coated with a monoclonal antibody of the invention, optionally freeze-dried or concentrated solutions of a monoclonal antibody of the invention and/or a radiolabelled derivative thereof or radiolabelled R-(-)-oxaprotiline, R-(-)-oxaprotiline or standard solutions of R-(-)-oxaprotiline, buffer solutions and, optionally, polypeptides, detergents and adjuncts for preventing non-specific adsorption and aggregate formation, pipettes, reaction vessels, calibration curves, instruction manuals and the like.

Test kits according to the invention for an enzyme immunoassay comprise, for example, a suitable carrier, e.g. microtitre plates or nitrocellulose sheets, uncoated or coated with a monoclonal antibody of the invention or with an R-(-)-oxaprotiline conjugate, optionally freeze-dried or concentrated solutions of a monoclonal antibody of the invention and/or of an enzyme-labelled monoclonal or polyclonal antibody to R-(-)-oxaprotiline or to a first antibody recognizing R-(-)-oxaprotiline, or of enzyme-labelled R-(-)-oxaprotiline, enzyme substrates in solid or dissolved form, R-(-)-oxaprotiline or standard solutions of R-(-)-oxaprotiline, buffer solutions and, optionally, polypeptides, detergents and adjuncts, pipettes, reaction vessels, calibration curves, colour scale tables, instruction manuals and the like.

Test kits according to the invention for a chemiluminescence test comprise, for example, a suitable carrier, e.g. polystyrene tubes, optionally freeze-dried or concentrated solutions of a monoclonal antibody of the invention and of a polyclonal antibody recognizing the antibody of the invention conjugated to a chemiluminescent marker, e.g. acridinium ester, solutions of a compound triggering the emission of light, e.g. $H_2O_2$ and NaOH, buffer solutions, R-(-)-oxaprotiline or standard solutions containing R-(-)-oxaprotiline, and, optionally, polypeptides, detergents and adjuncts, pipettes, reaction vessels, instruction manuals and the like.

A special embodiment of a test kit according to the invention is an immunostrip (dipstick) made of porous solid phase material, e.g. nitrocellulose, impregnated with an unlabelled monoclonal antibody of the invention in a detection zone. The antibody is firmly immobilized. The test solution, e.g. whole blood, plasma, serum or urine, containing R-(-)-oxaprotiline or its beta-glucuronide, is applied to one portion of the test strip and is allowed to permeate through the strip material, usually with the aid of an eluting solvent such as water. Thus, the sample progresses into or through the detection zone. The extent to which R-(-)-oxaprotiline or R-(-)-oxaprotiline beta-glucuronide of the sample becomes bound in that zone can be determined with the aid of labelled binding reagents, which can also be incorporated in the test strip or applied thereto subsequently. The labelled binding reagent is freely mobile within the porous carrier when in the moist state, and is bound in the detection zone during migration. The labelled binding reagent is a specific binding partner for R-(-)-oxaprotiline or its beta-glucuronide, e.g. a labelled monoclonal antibody of the invention which recognizes a different epitope of R-(-)-oxaprotiline or R-(-)-oxaprotiline beta-glucuronide than the immobilized antibody. In the alternative, the labelled binding reagent is R-(-)-oxaprotiline or its beta-glucuronide itself which has been conjugated with a label. The label produces a readily detectable signal, for example a color formation or color change, in the detection zone. It is a direct label, such as a metallic (e.g. gold) or dye solution, or an indirect label, such as an enzyme, e.g. alkaline phosphatase or horseradish peroxidase. Labelling is carried out according to known methods. Optionally, the immunostrip may be contained in an analytical test device comprising a hollow casing constructed of moisture-impervious solid material.

A typical example is an immunostrip using the principle of a competitive immunoassay. A strip of porous solid phase material, preferably nitrocellulose, is impregnated in a first zone with R-(-)-oxaprotiline or R-(-)-oxaprotiline beta-glucuronide and a label, for example a direct label such as a coloured dye or an indirect label such as an enzyme, e.g. alkaline phosphatase. The labelled R-(-)-oxaprotiline or R-(-)-oxaprotiline beta-glucuronide is retained in the first zone while the porous material is in the dry state but is free to migrate when the porous material is moistened, for example by the application of an aqueous liquid test sample. In a second (detection) zone, which is spatially distinct from the first zone, a monoclonal antibody of the invention is firmly immobilized on the porous material so that it is not free to migrate through the porous material in the moist state. After application of the test sample, e.g. whole blood, plasma, serum or urine containing an unknown amount of R-(-)-oxaprotiline or R-(-)-oxaprotiline beta-glucuronide to be determined, the R-(-)-oxaprotiline or R-(-)-oxaprotiline beta-glucuronide of the test sample and the labelled R-(-)-oxaprotiline or R-(-)-oxaprotiline beta-glucuronide migrate through the porous material and compete for the binding sites of the immobilized monoclonal antibody in the detection zone. The amount of immune complex formed in the detection zone is determined by detection of the label, for example by detection of the colour generated in the detection zone by eye or an optical measuring device, either directly when a dye is used as label or after addition of an appropriate substrate solution when an enzyme is used as label.

The monoclonal antibodies of the invention or derivatives thereof can also be used in any known modification of an immunostrip employing the principle of immunoassays such as radioimmunoassays, chemiluminescence immunoassays, and the like, as described hereinbefore.

The monoclonal antibodies of the invention are also useful in the therapy of an overdose of R-(-)-oxaprotiline. The monoclonal antibodies are applied in the form of pharmaceutical preparations that comprise a therapeutically effective amount of the monoclonal antibodies optionally together or in admixture with inorganic or organic, solid or liquid, pharmaceutically acceptable carriers.

The pharmaceutical preparations according to the invention are those for parenteral, e.g. intranasal, intramuscular, subcutaneous or intravenous, administration to warm-blooded animals, for example humans. Depending on the intended method of administration, the pharmaceutical preparations may be in unit dose form, for example in ampoules, vials, suppositories, dragées, tablets, capsules or nasal sprays in liquid or solid form.

The amount of the therapeutically effective compounds to be administered depends on the condition of the warm-blooded animal, for example man, such as the body weight and the general condition, on the amount of R-(-)-oxaprotiline which has led to the symptoms of a toxicosis, and also on the mode of

administration, and is carried out in accordance with the assessment of the physician giving the treatment. The effective dose is in the order of magnitude of from 1 to 100 mg per kg of body weight and is applied in a single dose or in several doses within one, two or three days.

The pharmaceutical preparations according to the invention comprise the customary inorganic or organic, solid or liquid pharmaceutically acceptable carriers, optionally together with other therapeutically effective compounds and/or adjuncts. There are preferably used solutions or suspensions of the antibodies, especially isotonic aqueous solutions or suspensions, or also lyophilized preparations which are dissolved in water shortly before use. The pharmaceutical preparations may be sterilized and/or contain preservatives, stabilizers, wetting agents, emulsifiers, solubilizers, viscosity-increasing substances, salts for regulating the osmotic pressure and/or buffers, and also other proteins, for example human serum albumin or human blood plasma preparations.

The following examples illustrate the invention, but do not limit it to any extent.

The abbreviations used in the examples have the following meaning:

BSA bovine serum albumin
BSS balanced salt solution
DMF N,N-dimethylformamide
FCS fetal calf serum
HAT hypoxanthine/aminopterin/thymidine
Ig immunoglobulin
PBS phosphate buffered saline

## Example 1 Preparation of hybridoma cell lines secreting monoclonal antibodies to R-(-)-oxaprotiline

### 1.1 Preparation of immunogenic conjugates of R-(-)-oxaprotiline

The immunogen used to elicit antibodies is an immunogenic conjugate of R-(-)-oxaprotiline with succinylated BSA which is prepared by the following procedures.

For the preparation of succinylated BSA, 1 g of BSA (Sigma) is dissolved in 50 ml of 100 mM PBS pH 7. 1 g of succinic anhydride is added in portions while maintaining a pH between 6.7 and 7.3. The mixture is dialysed four times against distilled water at 4° C for 6 h, then lyophilized.

Then, the succinylated BSA is incubated with 33 mg R-(-)-oxaprotiline prepared according to EP 0 014 433 and 84.3 mg N-ethyl-N′-(3-dimethylaminopropyl)-carbodiimide for 2 h at room temperature. The mixture is exhaustively dialysed against distilled water to eliminate the excess of reagents. The obtained R-(-)-oxaprotiline-BSA conjugate is lyophilized.

### 1.2 Immunization of Balb/c mice

Balb/c mice aged 5-6 weeks are injected each subcutaneously and intraperitoneally with 30 µg of the R-(-)-oxaprotiline-BSA conjugate of example 1.1 in complete Freund's adjuvant (300 µl). Four weeks later 30 µg of the conjugate in incomplete Freund's adjuvant are injected intraperitoneally and subcutaneously. After another 3 weeks, 30 µg of the conjugate in phosphate buffered saline are injected intraperitoneally. After one to two further weeks, blood of the mice is withdrawn, and the serum tested by the immunoassay of example 1.4.

An additional booster injection of 30 µg R-(-)-oxaprotiline-BSA conjugate in 0.9 % saline is given intravenously 2 to 3 weeks later. Three days later the spleens of mice whose serum is positive in the immunoassay of example 1.4 are excised.

### 1.3 Cell fusion

The fusion procedure follows a modification of the general protocol described by G. Kohler and C. Milstein (Nature 256, 495, 1975). Pellets of 10 x $10^7$ spleen cells and 1-2 x $10^7$ myeloma cells Sp2/O-Ag14 (Shulman et al., Nature 276, 269, 1978) or PAI (Stocker et al., Hoffmann-LaRoche Research Disclosure No. 21713, 1982) are mixed in the presence of 41 % polyethylene glycol (PEG 4000, Merck), washed in RPMI 1640 medium and resuspended in RPMI 1640 containing 10 % FCS at 2 x $10^6$ cells per ml. The cells are

distributed in 0.5 ml aliquots in the wells of 24 well microtitre plates (Costar). At day 1, 8, 10 and 12, fresh medium containing hypoxanthine, aminopterin und thymidine (HAT) is added. At day 15 the HAT medium is replaced by medium HT without aminopterin. At day 29 the surviving hybridoma cells are cultivated in RPMI 1640/10 % FCS. The supernatants of growing clones are tested for the presence of antibodies to R-(-)-oxaprotiline by the immunoassay of example 1.4. Positive hybridomas are cloned by limiting dilution in the presence of syngeneic peritoneal mouse macrophages (10,000 cells per well) in microtitre plates. The hybridoma cells can be grown in culture, frozen at -80° C or in liquid nitrogen and then reactivated.

1.4 Antibody screening with an enzyme linked immunosorbent assay (ELISA)

The growing hybridomas are tested for the presence of anti-R-(-)-oxaprotiline antibodies by an enzyme linked immunosorbent assay (ELISA).

The wells of a microtitre plate (Dynatech MicrotestTM) are coated with a conjugate of R-(-)-oxaprotiline with mucin in order to avoid any interference with antibodies to BSA. The R-(-)-oxaprotiline-mucin conjugate is prepared by the following procedure.

28 mg mucin from bovine submaxillary glands (type I, Sigma M-4503) are dissolved in 5 ml water. 85 $\mu$l 0.1 sodium periodate are added, the solution is left at room temperature for 1 h, and the mixture is dialysed against water. 125 $\mu$l of 1M sodium cyanoborohydride are added, followed by 6.3 mg R-(-)-oxaprotiline dissolved in 1 ml water and 0.5 ml 1N HCl. A precipitate is formed. The mixture is kept at 70° C for 1.5 h and dialysed overnight against water and 4M guanidinium chloride.

The conjugate solution (0.66 mg/ml) is diluted 1000-fold in coating buffer pH 9.8 (477 mg sodium carbonate, 897 mg sodium hydrogen carbonate, 60 mg sodium azide, 300 ml distilled water) and incubated in the wells of a microtitre plate for 2 days at 4° C. The wells are washed three times with PBS containing Tween 20 (Sigma, 0.1 %). Residual adsorption sites on the plate are saturated by incubation with 100 $\mu$l of PBS/Tween 20 for 1 h. 100 $\mu$l of a solution to be tested for the presence of antibodies to R-(-)-oxaprotiline are incubated in the wells for 1 h at room temperature. After washing three times with PBS/Tween 20, an alkaline phosphatase conjugated anti-mouse IgG antiserum (Sigma) is added in a 1000-fold dilution for 1 h at 37° C. The microtitre plate is washed five times with PBS/Tween 20, and the enzyme-conjugated bound antiserum developed with an alkaline phosphatase substrate consisting of disodium p-nitrophenylphosphate (1 mg/ml; Sigma) in diethanolamine buffer pH 9.8. The substrate gives a yellow coloration which is measured with a colorimeter (Titertek Multiskan) at a wave length of 405 nm after 60 min.

To make sure that bound antibodies recognizing the R-(-)-oxaprotiline-mucin conjugate are directed against the hapten and not against the bond between mucin and R-(-)-oxaprotiline or a special conformation of bound R-(-)-oxaprotiline, an inhibition test of free R-(-)-oxaprotiline is performed as described in example 3.4.

The hybridomas with the respective designation 1228P54.6 and 1228P63.1 (fusion partner: cell line PAI) as well as 1219S59.11, 1219S78.6, 1220S36.6, 1225S93.2 and 1226S31.3 (fusion partner: cell line Sp2/O-Ag14) are chosen for further characterization. The monoclonal antibodies secreted by these hybridomas are designated by the prefix "MAb" and the number of the respective hybridoma, e.g. MAb 1219S59.11.

Example 2: Isolation and purification of monoclonal antibodies

Balb/c mice 4-6 weeks of age are pretreated intraperitoneally with 0.3 ml pristane (Aldrich). 1-3 weeks later, 1-2x$10^6$ cloned hybridoma cells in 0.2 ml BSS and 0.2 ml pristane are inoculated intraperitoneally. After 8-10 days ascitic fluid is collected, centrifuged at 800 x g and stored at -20° C.

Defrosted ascitic fluid is centrifuged at 50,000 x g for 60 min. A fat layer floating on the surface is carefully removed, and the protein concentration is adjusted to a concentration of 10 mg/ml. Crude immunoglobulin is precipitated by dropwise addition of 0.9 volume equivalents of saturated ammonium sulphate at 4° C, then dissolved in 20 mM TrisHCl and 50 mM NaCl pH 7.9 and dialysed three times against this buffer. The solution is diluted with 20 mM TrisHCl pH 7.9 at 1:1 (v/v) and antibodies are purified by chromatography on DEAE cellulose (Whatman). Fractions positive in the immunoassay of example 1.4 are combined and dialysed against 0.5 M PBS pH 7.2.

It is also possible to obtain smaller amounts of monoclonal antibodies (ca. 1 mg) by in vitro propagation. The hybridoma cells are cultivated at physiological temperature (ca. 37° C) in culture medium (RPMI 1640 supplemented with 10 % FCS) in plastic flasks (Costar) to a cell density of 5 x $10^5$-$10^6$ cells/ml. The complete preculture is transferred into Bellco spinner-bottles graduated to a volume of 3000 ml. Culture

medium is added to give a final volume of 1500 ml. The culture is stirred for two to three days at 30 rpm in an air atmosphere enriched with 5 % $CO_2$ at physiological temperature. The volume of the culture is increased to 3000 ml with more culture medium. The culture is cultivated for another 7-10 days using the above culturing conditions. The medium containing the cells is centrifuged at 1000 x g for 20 min at 4° C. The supernatant is filtered (0.2 µm pore size) and concentrated under sterile conditions. The crude immunoglobulin is obtained by addition of saturated ammonium sulphate, then purified as described hereinbefore.

## Example 3: Characterization of the anti-R-(-)-oxaprotiline monoclonal antibodies

### 3.1 Determination of class and subclass

The isotype of the monoclonal antibodies is determined by ELISA analysis with rabbit antisera to mouse Ig classes and subclasses (Biorad Mouse Typer™ Sub Isotyping Kit).

All monoclonal antibodies are of IgG1 isotype with the exception of MAb 1219S78.6 which is IgG2b.

### 3.2 Inhibition of monoclonal antibody binding to R-(-)oxaprotiline by therapeutic drugs and biogenic amines

The capacity of a number of therapeutic drugs some of which are used in combination with antidepressants and of biogenic amines to inhibit the binding of the monoclonal antibodies of the invention to the antigen is determined in a two-step inhibition enzyme immunoassay to establish the selectivity of the monoclonal antibodies to the tested compounds.

The assay is carried out as described in the following. A defined concentration of the monoclonal antibody to be tested (equivalent to the amount of antibody resulting in an extinction 20fold above controls in the ELISA of example 1.4) in 60 µl PBS/Tween 20 (0.05 %) is preincubated overnight in uncoated 96-well microtitre plates with samples containing 60 µl of distinct concentrations of each of the various compounds to be tested (1 µM-150 pM) in PBS/Tween 20 (0.05 %). The incubation mixture is then transferred to R-(-)-oxaprotiline-mucin coated microtitre plates and the relative concentration of antibody not engaged in immunocomplexes with the soluble compound is determined as described in example 1.4.

The relative binding affinity of the monoclonal antibodies to the soluble compounds is expressed as $IC_{50}$ (inhibition concentration), i.e. the concentration of the compound which inhibits the binding of the monoclonal antibody to the solid phase antigen in the range of 50 %. The results for the monoclonal antibody MAb 1226S31.3 are given in Table 1.

Table 1 Inhibition of binding of Mab 1226S31.3 to surface-bound R-(-)-oxaprotiline-mucin conjugate by therapeutic drugs and by biogenic amines

| brand name | generic name | systematic name | structural formula | $IC_{50}$* (nM) | clinical use |
|---|---|---|---|---|---|
| Pertofran | Desipramine | 10,11-dihydro-N-methyl-5H-dibenzo[b,f]azepine-5-propanamine | 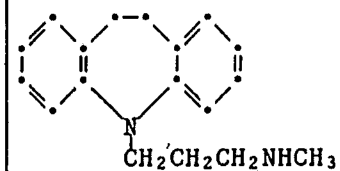 | 406 | antidepressant |
| | Imipramine | 10,11-dihydro-N,N-dimethyl-5H-dibenzo[b,f]azepine-5-propanamine | 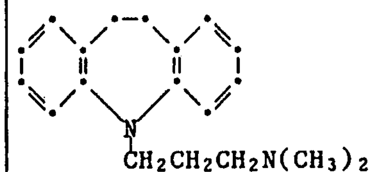 | 855 | antidepressant |
| Laroxyl | Amitriptyline | 3-(10,11-dihydro-5H-dibenzo[a,d]cyclohepten-5-ylidene-N,N-dimethyl-1-propanamine | 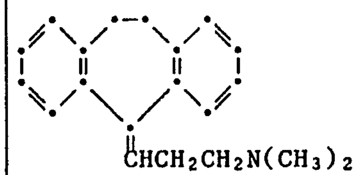 | 548 | antidepressant |
| | Chlorpromazine | 2-chloro-N,N-dimethyl-10H-phenothiazine-10-propanamine | 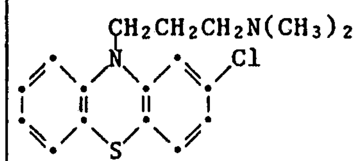 | 659 | neuroleptic |

Table 1 continued

| brand name | generic name | systematic name | structural formula | $IC_{50}$* (nM) | clinical use |
|---|---|---|---|---|---|
| Tegretol | Carbamazepine | 5H-dibenzo[b,f]azepine-5-carboxamide | 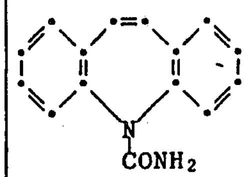 | -- | anticonvulsant |
| Haldol | Haloperidol | 4-[4-(4-chlorophenyl)-4-hydroxy-1-piperidinyl]-1-(4-fluorophenyl)-1-butanone | 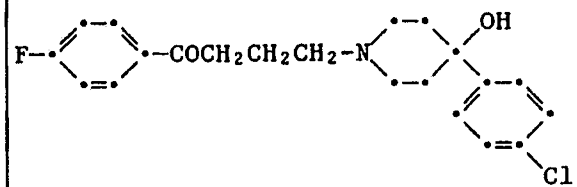 | -- | neuroleptic |
| Librium | Chlordiazepoxide | 7-chloro-N-methyl-5-phenyl-3H-1,4-benzodiazepine-2-amine-4-oxide | 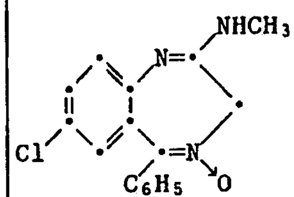 | -- | sedative tranquilizer |
| Inderal | Propranolol | 1-[(1-methylethyl)amino]-3-(1-naphthalenyloxy)-2-propanol | 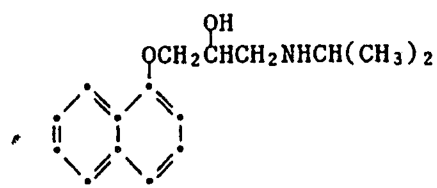 | -- | antiarrhythmic agent β-blocker |

Table 1 continued

| brand name | generic name | systematic name | structural formula | $IC_{50}$* (nM) | clinical use |
|---|---|---|---|---|---|
| | Epinephrin | 4-[1-hydroxy-2-(methyl-amino)-ethyl]-1,2-benzene-diol | 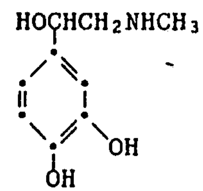 | -- | - |
| | L-Norepinephrin | 4-[2-amino-1-hydroxy-ethyl]-1,2-benzenediol | 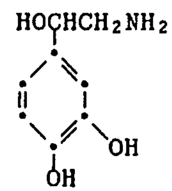 | -- | - |
| | Histamine | 1H-imidazole-4-ethane-amine | 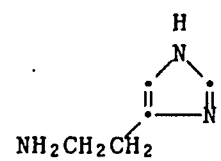 | -- | - |

Symbols: -- no inhibition

*$IC_{50}$ of R-(-)-oxaprotiline: 6.7 nM (see Table 2B)

The tricyclic drugs with an alkylamino side chain show weak inhibitory activity whereas none of the other tested compounds are inhibiting at any concentration tested.

3.3 Inhibition of monoclonal antibody binding to R-(-)-oxaprotiline by free R-(-)-oxaprotiline and by structural analogues

The capacity of free R-(-)-oxaprotiline and of a number of compounds which are structurally analogous to R-(-)-oxaprotiline to inhibit the binding of the monoclonal antibodies of the invention to the antigen is determined in an inhibition enzyme immunoassay essentially as described in example 3.2. The MAbs are preincubated with free R-(-)-oxaprotiline and with various structural analogues, respectively, instead of with the test compounds of example 3.2. The tested structural analogues are listed in Table 2A.

Preincubation with free R-(-)-oxaprotiline is carried out to investigate possible conformational changes of R(-)-oxaprotiline upon binding to a solid surface and gives a general index of the binding affinity of the monoclonal antibodies to the antigen.

The $IC_{50}$ values and the relative affinities derived therefrom for the monoclonal antibodies MAb 1219S59.11, MAb 1219S78.6, MAb 1225S93.2, MAb 1226S31.3 and MAb 1228P63.1 are shown in Table 2B.

Table 2A Compounds/structural analogues tested in the inhibition test

| compound no. | systematic name [generic name] | structural formula |
|---|---|---|
| 1 | R-(-)-1-(2-hydroxy-3-methylamino-propyl-dibenzo[b,e]bicyclo-[2.2.2]octadiene-hydrochloride [R-(-)-oxaprotiline] | HCl 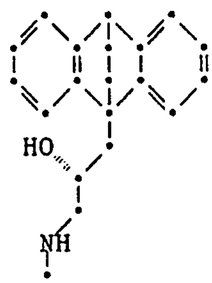 |
| 2 | S-(+)-1-(2-hydroxy-3-methylamino-propyl)-dibenzo[b,e]bicyclo-[2.2.2]octadiene-hydrochloride [S-(+)-oxaprotiline] | HCl 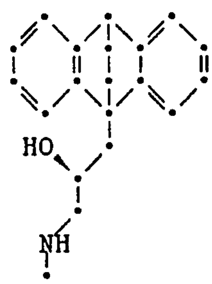 |
| 3 | 1-(2-hydroxy-3-methyl-amino-propyl)-dibenzo-[b,e]bicyclo[2.2.2]-octadiene-hydro-chloride [racemic oxaprotiline] | HCl 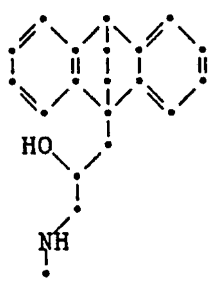 |
| 4 | R-(-)-1-(2-glucuronyl-3-methylamino-propyl)-dibenzo[b,e]bicyclo-[2.2.2]octadiene [R-(-)-oxaprotiline-β-glucuronide] | 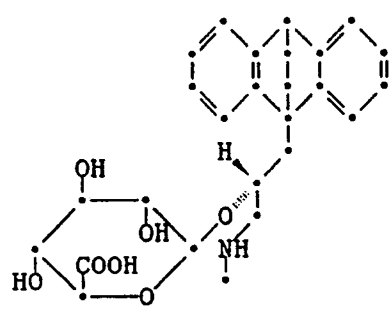 |
| 5 | S-(+)-1-(2-glucuronyl-3-methylamino-propyl)-dibenzo[b,e]bicyclo-[2.2.2]octadiene [S-(+)-oxaprotiline-β-glucuronide] | 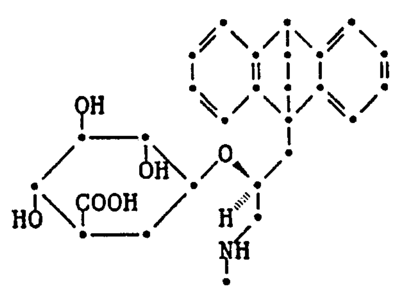 |

Table 2A, continued

| compound no. | systematic name [generic name] | structural formula |
| --- | --- | --- |
| 6 | 1-(3-methylamino-propyl)-dibenzo[b,e]-bicyclo[2.2.2]-octadiene-hydro-chloride [maprotiline] | HCl 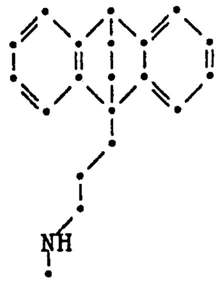 |
| 7 | 1-(3-amino-2-hydroxy-propyl)-dibenzo[b,e]-bicyclo[2.2.2]-octadiene-hydro-chloride [des-N-methyl-oxa-protiline] | HCl 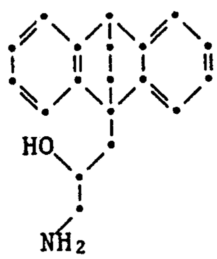 |
| 8 | 1-(3-dimethylamino-2-hydroxy-1-propyl)-dibenzo[b,e]bicyclo-[2.2.2]octadiene-methanesulfonate [N,N-dimethyl-oxa-protiline] | 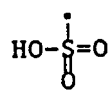 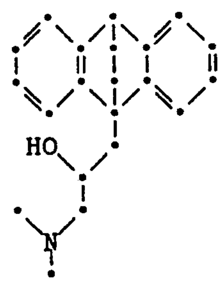 |
| 9 | 1-(2-methoxy-3-methylamino-propyl)-dibenzo[b,e]bicyclo-[2.2.2]octadiene-methanesulfonate [2-O-methyl-oxaprotiline] | 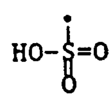 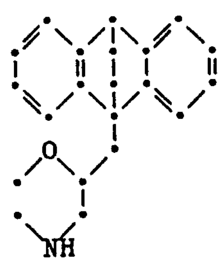 |
| 10 | 1-(2-hydroxy-3-methyl-amino-2-methyl-propyl)-dibenzo[b,e]bicyclo-[2.2.2]octadiene-hydrochloride [2-methyl-oxaprotiline] | HCl 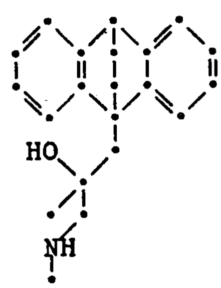 |

Table 2A, continued

| compound no. | systematic name [generic name] | structural formula |
|---|---|---|
| 11 | 1-(2-acetoxy-3-methyl-amino-propyl)-dibenzo[b,e]bicyclo-[2.2.2]octadiene-hydrochloride-semi-hydrate [2-acetoxy-oxa-protiline] | 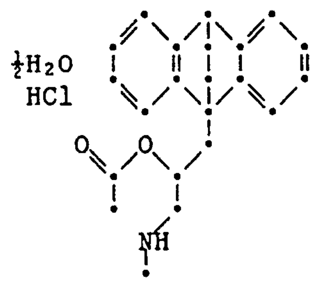 |
| 12 | 2-(dibenzo[b,e]bicyclo-[2.2.2]octadienyl-1-methyl)-morpholine-hydrochloride [morpholino-maprotiline] | 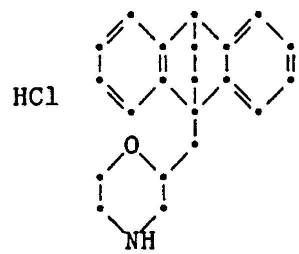 |
| 13 | 3-methyl-5-(dibenzo-[b,e]bicyclo[2.2.2]-octadienyl-1-methyl)-oxazolidine-methane-sulfonate [oxazolidino-maprotiline] | 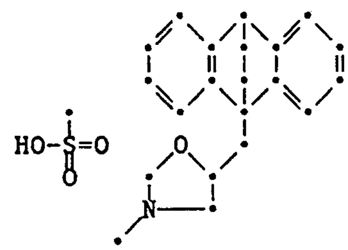 |
| 14 | 1-(2-hydroxy-3-methyl-amino-propyl)-10-chloro-dibenzo[b,e]-bicyclo-[2.2.2]-octadiene-hydrochloride [10-chloro-oxa-protiline] | 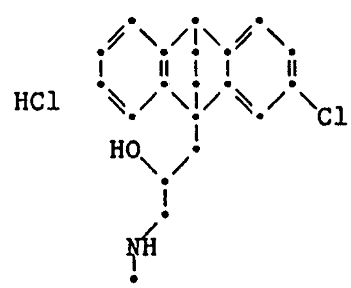 |
| 15 | 1-(2-hydroxy-3-dimethylamino-propyl]-10-chlorodibenzo[b,e]-bicyclo[2.2.2]-octadiene-cyclamate [10-chloro-N,N-dimethyl-oxaprotiline] | 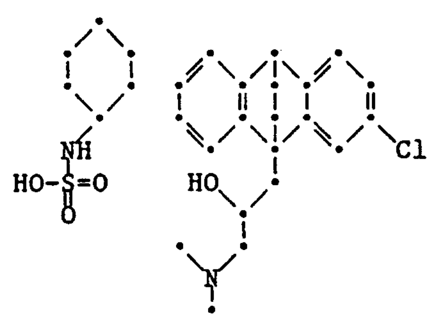 |

Table 2A, continued

| compound no. | systematic name [generic name] | structural formula |
|---|---|---|
| 16 | 1-(2-hydroxy-3-methyl-amino-propyl)-10-tri-fluoromethyl-dibenzo-[b,e]bicyclo[2.2.2]-octadiene-hydro-chloride-hydrate | $H_2O$ HCl 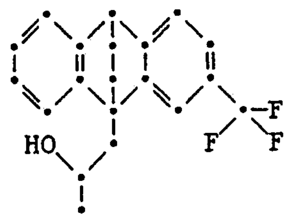 |
| 17 | 1-(2-hydroxy-3-di-methylamino-propyl)-10-trifluoromethyl-dibenzo[b,e]bicyclo-[2.2.2]octadiene-hydrochloride [10-trifluoromethyl-N,N-dimethyl-oxa-protiline] | HCl 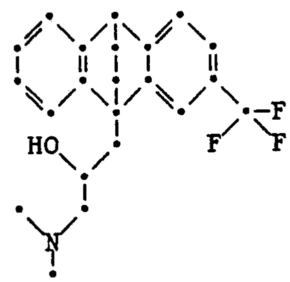 |

Table 2B

Inhibition of monoclonal antibody binding to surface bound R-(-)-oxaprotiline-mucin conjugate by structural analogues

| compound/structural analogue (see Table 2B) | MAb 1219S59.11 | | MAb 1219S78.6 | | MAb 1225S93.2 | | MAb 1226S31.3 | | MAb 1228P63.1 | |
|---|---|---|---|---|---|---|---|---|---|---|
| | $IC_{50}$(nM) | rel. affinity | $IC_{50}$(nM) | rel. affinity | $IC_{50}$(nM) | rel. affinity | $IC_{50}$(nM) | rel. affinity | $IC_{50}$(nM) | rel. affinity |
| 1 | 0.29 | 1 | 0.305 | 1 | 0.37 | 1 | 6.7 | 1 | 0.75 | 1 |
| 2 | 134.0 | 462.0 | 38.5 | 126.2 | 0.85 | 2.3 | ~4.0 | ~0.6 | 4.9 | 6.5 |
| 3 | 0.75 | 2.6 | 0.515 | 1.7 | 0.475 | 1.3 | 3.09 | 0.5 | 1.715 | 2.3 |
| 4 | 71.0 | 244.8 | 70.0 | 229.5 | 91.0 | 245.9 | 27.5 | 4.1 | 51.0 | 68.0 |
| 5 | >1000.0 | >3448 | >1000.0 | >3279 | 159.0 | 429.7 | 353.0 | 52.7 | 422.0 | 562.7 |
| 6 | 2.55 | 8.8 | 0.315 | 1.03 | 0.44 | 1.2 | 1.0 | 0.2 | 1.05 | 1.4 |
| 7 | 107.0 | 368.9 | 76.0 | 249.2 | 0.75 | 2.0 | 2.375 | 0.4 | 1.65 | 2.2 |
| 8 | 0.775 | 2.7 | 0.9 | 3.0 | 0.925 | 2.5 | 5.85 | 0.9 | 1.15 | 1.5 |
| 9 | 51.0 | 175.9 | 31.0 | 101.6 | 2.35 | 6.4 | 12.0 | 1.8 | 5.15 | 6.9 |
| 10 | 9.0 | 31.0 | 1.45 | 4.8 | 2.55 | 6.9 | 2.1 | 0.3 | 4.9 | 6.5 |
| 11 | 1.3 | 4.5 | 1.0 | 3.3 | 0.715 | 1.9 | 3.575 | 0.5 | 3.35 | 4.5 |
| 12 | 50.5 | 174.1 | ~15.0 | ~49.2 | 2.35 | 6.4 | 2.1 | 0.3 | 2.35 | 3.1 |
| 13 | 0.55 | 1.9 | 0.48 | 1.6 | 0.58 | 1.6 | 4.45 | 0.7 | 1.2 | 1.6 |
| 14 | ~900.0 | ~3103.4 | ~700.0 | ~2295.1 | 10.4 | 28.1 | 127.5 | 19.0 | 27.5 | 36.7 |
| 15 | 36.0 | 124.1 | 52.0 | 170.5 | 27.5 | 74.3 | 50.0 | 7.5 | 12.5 | 16.7 |
| 16 | >1000.0 | >3448 | >1000.0 | >3279 | 93.0 | 251.4 | >1000.0 | >149 | >1250.0 | >1667 |
| 17 | >1000.0 | >3448 | >1000.0 | >3279 | 232.5 | 628.4 | >1000.0 | >149 | >1250.0 | >1667 |

MAb 1219S59.11 is the antibody showing the highest degree of selectivity and sensitivity for R-(-)-oxaprotiline. It expresses stereospecificity and crossreacts only moderately with most analogues. MAb 1219S78.6 reacts in a similar manner although less distinctly. MAbs 1225S93.2, 1226S31.3 and 1228P63.1 hardly discriminate between the two oxaprotiline enantiomers. Even some modifications of the common tetracyclic ring structure are recognized. Glucuronidation of the hydroxyl group decreases the binding capacity of all tested antibodies to various degrees. Considering the high urine concentrations of these metabolites, however, the sensitivity of the MAbs is still sufficient to determine the compound, e.g. for patient compliance.

Example 4: Competitive chemiluminescence immunoassay for the determination of R-(-)-oxaprotiline in serum or R-(-)-oxaprotiline beta-glucuronide in urine

The monoclonal antibodies of the invention can be used for the qualitative and/or quantitative determination of R-(-)-oxaprotiline in human serum or R-(-)-oxaprotiline beta-glucuronide in urine in a competitive chemiluminescence immunoassay described in the following.

4.1 Preparation of a conjugate of R-(-)-oxaprotiline succinate with acridinium ester

In a first step, R-(-)-oxaprotiline succinate is prepared as described in the following. 204 mg R-(-)-oxaprotiline•HCl are dissolved in 3 ml methanol and 1 ml water. 110 mg succinic anhydride are added with stirring and adjusting the pH to neutral with 1N NaOH. After 1 hour, the solution is acidified to pH 3 with HCl and diluted with water to a total volume of 25 ml. After cooling on ice, the precipitate is filtered off and dried (yield 95 %).

Then, 79 mg of R-(-)-oxaprotiline succinate are dissolved in 0.5 ml dioxane, 23 mg N-hydroxysuccinimide in 0.3 ml dioxane and 42 mg dicyclohexylcarbodiimide in 0.3 ml dioxane. The solution is reacted for 2 hours at room temperature. 4 ml ethyl acetate are added, the precipitate is filtered off and the solution is extracted with 2 ml of an ice cold 10 % $NaHCO_3$ solution and 2 ml water. The organic phase is dried with sodium sulphate and filtered off under nitrogen.

The thus prepared N-hydroxysuccinimide ester of R-(-)-oxaprotiline succinate is in a third step conjugated to lysozyme. One volume of a 2 mM solution of activated ester and one volume of a 2 mM solution of acridinium ester in DMF are incubated with 5 volumes of a 0.2 mM solution of lysozyme in 0.1 M phosphate buffer and 0.15 M sodium chloride pH 8 for 2 hours. The conjugate is purified by chromatography on Biogel P-6.

4.2 Immunoassay with bound monoclonal antibody

100 µl of a monoclonal anti-R-(-)-oxaprotiline antibody (10 µg/ml) are incubated in polystyrene tubes for 1 h at room temperature. After washing three times with PBS, residual adsorption sites are saturated with PBS containing gelatin (2.5 mg/ml) and Tween 20TM (Sigma, 0.1 %) for 1 h. Serum and urine samples to be tested are supplemented with 0.8 µg/ml of the conjugate of R-(-)-oxaprotiline succinate with acridinium ester and lysozyme of example 4.1. The samples are incubated in the polystyrene tubes coated with the MAb for 1 h at room temperature. The tubes are washed with PBS, and the bound conjugate is detected by addition of hydrogen peroxide/sodium hydroxide, which triggers the luminescence response, and measuring the light emission in a Ciba-Corning Magic Lite II luminometer.

4.3 Test kit

A test kit for the immunoassay of example 4.2 contains:

polystyrene tubes
10 ml conjugate of R-(-)-oxaprotiline succinate with acridinium ester and lysozyme, 20 µg/ml in PBS
10 ml anti-R-(-)-oxaprotiline monoclonal antibody, 70 µg/ml in PBS 100 ml PBS containing gelatin (2.5 mg/ml) and Tween 20TM (0.1 %)
10 mg R-(-)-oxaprotiline

instruction leaflet

Example 5 Competitive immunoassay on immunostrip or dipstick for the detection of R-(-)-oxaprotiline beta-glucuronide in urine

R-(-)-oxaprotiline beta-glucuronide can be detected in urine in a competitive enzyme immunoassay on an immunostrip or a dipstick.

A monoclonal antibody of the invention (2 mg/ml in PBS pH 7.4) is applied to 1 cm x 5 cm strips of nitrocellulose (8 µm; Schleicher and Schuell) with the help of a microsyringe in a restricted detection zone. The applied material is allowed to dry for 1 h at room temperature, excess binding sites on the nitrocellulose are blocked with polyvinyl alcohol (1 % w/v in 20 mM TrisHCl pH 7.4) for 30 min at room temperature, the sheets are thoroughly rinsed with distilled water and allowed to dry for 30 min at 30 °C.

A conjugate of R-(-)-oxaprotiline beta-glucuronide with alkaline phosphatase is prepared and applied to the nitrocellulose sheets in a distinct zone below the immobilized antibody on top of a sublayer of sucrose (60 % w/v in distilled water). The strips are allowed to dry.

The assay is performed by applying fresh urine sample (100 µl) to the application zone of the immunostrip. Each sample is allowed to run for 5 min on the strip. Then, substrate solution is added and the colour generated at the detection zone is read by eye or a light reflectometer.

Alternatively, a conjugate of R-(-)-oxaprotiline beta-glucuronide with a covalently bound dyestuff is used instead of the alkaline phosphatase conjugate. In this case, no substrate addition is necessary for the generation of colour at the detection zone.

Example 6 Pharmaceutical preparation for parenteral use

2.0 mg of a monoclonal antibody of the invention are dissolved in 50 ml physiological saline. The solution is passed through a bacteriological filter and the filtrate filled in 10 ampoules (5 ml) under aseptic conditions. The ampoules are preferentially stored in the cold, e.g. at -20 °C.

## Claims

1. A monoclonal antibody directed against R-(-)-oxaprotiline and/or R-(-)-oxaprotiline beta-glucuronide, and a derivative thereof which retains its specificity for the antigenic determinants of said compounds.

2. A monoclonal antibody and a derivative thereof as claimed in claim 1 directed against R-(-)-oxaprotiline.

3. A monoclonal antibody and a derivative thereof as claimed in claim 1 or 2 which selectively bind to R-(-)-oxaprotiline in the presence of other dibenzobicyclo[2.2.2]octadiene compounds which differ from R-(-)-oxaprotiline by chemical modifications of the tetracyclic ring structure and/or the aliphatic side chain.

4. A monoclonal antibody and a derivative thereof as claimed in any of the claims 1 to 3 which selectively bind to R-(-)-oxaprotiline in the presence of other therapeutic drugs potentially used in combination with R-(-)-oxaprotiline which lack the dibenzobicyclo[2.2.2]octadiene skeleton.

5. A monoclonal antibody and a derivative thereof as claimed in any of the claims 1 to 4 which selectively bind to R-(-)-oxaprotiline in the presence of S-(+)-oxaprotiline.

6. A monoclonal antibody as claimed in any of the claims 1 to 5 selected from the antibodies with the designation MAb 1219S59.11, MAb 1219S78.6, MAb 1220S36.6, MAb 1225S93.2, MAb 1226S31.3, MAb 1228P54.6 and MAb 1228P63.1, and a derivative thereof.

7. The monoclonal antibody as claimed in claim 6 with the designation MAb 1219S59.11, and a derivative thereof.

8. The monoclonal antibody as claimed in claim 6 with the designation MAb 1219S78.6, and a derivative thereof.

9. The monoclonal antibody as claimed in claim 6 with the designation MAb 1226S31.3, and a derivative thereof.

10. The monoclonal antibody as claimed in claim 6 with the designation MAb 1228P63.1, and a derivative thereof.

11. A derivative of a monoclonal antibody as claimed in any of the claims 1 to 10 which is a conjugate with an enzyme, a fluorescent marker, a chemiluminescent marker, a metal chelate, avidin, biotin, or the

like.

12. A derivative of a monoclonal antibody as claimed in any of the claims 1 to 10 which is radioactively labelled.

13. A derivative of a monoclonal antibody as claimed in any of the claims 1 to 10 which is a fragment.

14. A process for the preparation of monoclonal antibodies and derivatives thereof as claimed in any of the claims 1 to 13, characterized in that hybridoma cells secreting said monoclonal antibodies are multiplied in vitro or in vivo and, when required, the obtained monoclonal antibodies are isolated and/or converted into derivatives thereof.

15. A hybridoma cell line, characterized in that it secretes monoclonal antibodies as claimed in claim 1.

16. A hybridoma cell line as claimed in claim 15, characterized in that it is a hybrid of myeloma cells and B lymphocytes of a mammal immunized with an immunogenic R-(-)-oxaprotiline conjugate.

17. A hybridoma cell line as claimed in claim 15, characterized in that it is a hybrid of mouse myeloma cells and B lymphocytes of a syngeneic mouse immunized with an immunogenic R-(-)-oxaprotiline conjugate.

18. A hybridoma cell line as claimed in claim 15 selected from the hybridoma cell lines with the designation 1219S59.11, 1219S78.6, 1220S36.6, 1225S93.2, 1226S31.3, 1228P54.6 and 1228P63.1.

19. The hybridoma cell line as claimed in claim 18 with the designation 1219S59.11.

20. The hybridoma cell line as claimed in claim 18 with the designation 1219S78.6.

21. The hybridoma cell line as claimed in claim 18 with the designation S1226S31.3.

22. The hybridoma cell line as claimed in claim 18 with the designation 1228P63. 1.

23. A process for the preparation of hybridoma cell lines as claimed in claim 15, characterized in that a suitable mammal is immunized with an immunogenic R-(-)-oxaprotiline conjugate, antibody-producing cells of this mammal are fused with cells of a continuous cell line, the hybrid cells obtained in the fusion are cloned, and cell clones secreting the desired monoclonal antibodies are selected.

24. A method of determination of R-(-)-oxaprotiline and/or R-(-)-oxaprotiline beta-glucuronide, characterized in that a monoclonal antibody and/or a derivative thereof as claimed in claim 1 are used in an immunoassay.

25. A method as claimed in claim 24, characterized in that the immunoassay is a radioimmunoassay.

26. A method as claimed in claim 24, characterized in that the immunoassay is an enzyme immunoassay.

27. A method as claimed in claim 24, characterized in that the immunoassay is a chemiluminescence immunoassay.

28. A test kit for the qualitative and quantitative determination of R-(-)-oxaprotiline and/or R-(-)-oxaprotiline beta-glucuronide comprising monoclonal antibodies and/or derivatives thereof as claimed in claim 1.

29. A test kit according to claim 28 which is an immunostrip.

30. Pharmaceutical preparations comprising monoclonal antibodies and/or derivatives thereof as claimed in claim 1 and a pharmaceutical carrier.

Claims for the following Contracting States: ES, GR

1. A process for the preparation of a monoclonal antibody directed against R-(-)-oxaprotiline and/or R-(-)-oxaprotiline beta-glucuronide, and a derivative thereof which retains its specificity for the antigenic determinants of said compounds, characterized in that hybridoma cells secreting said monoclonal antibodies are multiplied in vitro or in vivo and, when required, the obtained monoclonal antibodies are isolated and/or converted into derivatives thereof.

2. A process according to claim 1 for the preparation of a monoclonal antibody and a derivative thereof directed against R-(-)-oxaprotiline.

3. A process according to claim 1 or 2 for the preparation of a monoclonal antibody and a derivative thereof which selectively bind to R-(-)-oxaprotiline in the presence of other dibenzobicyclo[2.2.2]octadiene compounds which differ from R-(-)-oxaprotiline by chemical modifications of the tetracyclic ring structure and/or the aliphatic side chain.

4. A process according to any of the claims 1 to 3 for the preparation of a monoclonal antibody and a derivative thereof which selectively bind to R-(-)-oxaprotiline in the presence of other therapeutic drugs potentially used in combination with R-(-)-oxaprotiline which lack the dibenzobicyclo[2.2.2]octadiene skeleton.

5. A process according to any of the claims 1 to 4 for the preparation of a monoclonal antibody and a derivative thereof which selectively bind to R-(-)-oxaprotiline in the presence of S-(+)-oxaprotiline.

6. A process according to any of the claims 1 to 5 for the preparation of a monoclonal antibody selected from the antibodies with the designation MAb 1219S59.11, MAb 1219S78.6, MAb 1220S36.6, MAb 1225S93.2, MAb 1226S31.3, MAb 1228P54.6 and MAb 1228P63.1, and a derivative thereof.

7. A process according to claim 6 for the preparation of the monoclonal antibody with the designation MAb 1219S59.11, and a derivative thereof.

8. A process according to claim 6 for the preparation of the monoclonal antibody with the designation MAb 1219S78.6, and a derivative thereof.

9. A process according to claim 6 for the preparation of the monoclonal antibody with the designation MAb 1226S31.3, and a derivative thereof.

10. A process according to claim 6 for the preparation of the monoclonal antibody with the designation MAb 1228P63.1, and a derivative thereof.

11. A process according to any of the claims 1 to 10 for the preparation of a derivative of a monoclonal antibody which is a conjugate with an enzyme, a fluorescent marker, a chemiluminescent marker, a metal chelate, avidin, biotin, or the like.

12. A process according to any of the claims 1 to 10 for the preparation of a derivative of a monoclonal antibody which is radioactively labelled.

13. A process according to any of the claims 1 to 10 for the preparation of a derivative of a monoclonal antibody which is a fragment.

14. A hybridoma cell line, characterized in that it secretes monoclonal antibodies directed against R-(-)-oxaprotiline and/or R-(-)-oxaprotiline beta-glucuronide.

15. A hybridoma cell line according to claim 14, characterized in that it is a hybrid of myeloma cells and B lymphocytes of a mammal immunized with an immunogenic R-(-)-oxaprotiline conjugate.

16. A hybridoma cell line according to claim 14, characterized in that it is a hybrid of mouse myeloma cells and B lymphocytes of a syngeneic mouse immunized with an immunogenic R-(-)-oxaprotiline conjugate.

17. A hybridoma cell line according to claim 14 selected from the hybridoma cell lines with the designation 1219S59.11, 1219S78.6, 1220S36.6, 1225S93.2, 1226S31.3, 1228P54.6 and 1228P63.1.

18. The hybridoma cell line according to claim 17 with the designation 1219S59.11.

19. The hybridoma cell line according to claim 17 with the designation 1219S78.6.

20. The hybridoma cell line according to claim 17 with the designation S1226S31.3.

21. The hybridoma cell line according to claim 17 with the designation 1228P63.1.

22. A process for the preparation of hybridoma cell lines according to claim 14, characterized in that a suitable mammal is immunized with an immunogenic R-(-)-oxaprotiline conjugate, antibody-producing cells of this mammal are fused with cells of a continuous cell line, the hybrid cells obtained in the fusion are cloned, and cell clones secreting the desired monoclonal antibodies are selected.

23. A method of determination of R-(-)-oxaprotiline and/or R-(-)-oxaprotiline beta-glucuronide, characterized in that a monoclonal antibody secreted by a hybridoma cell line according to claim 14 and/or a derivative thereof are used in an immunoassay.

24. A method as claimed in claim 23, characterized in that the immunoassay is a radioimmunoassay.

25. A method as claimed in claim 23, characterized in that the immunoassay is an enzyme immunoassay.

26. A method as claimed in claim 23, characterized in that the immunoassay is a chemiluminescence immunoassay.

27. A method of manufacture of pharmaceutical preparations comprising monoclonal antibodies secreted by a hybridoma cell line according to claim 14 and/or derivatives thereof, characterized in that the monoclonal antibodies and/or derivatives thereof are mixed with a pharmaceutical carrier.

European Patent Office

# EUROPEAN SEARCH REPORT

Application Number

EP 90 81 0260

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | MOLECULAR IMMUNOLOGY, vol. 25, no. 12, 1988, pages 1299-1308, Pergamon Press Plc, GB; L. CHOUCHANE et al.: "Stereospecific immuno-recognition of the tetracyclic anti-depressant oxaprotiline" * The whole article * ----- | 1-30 | C 12 P 21/08<br>C 12 N 5/12<br>A 61 K 39/395<br>G 01 N 33/577 |
| | | | **TECHNICAL FIELDS SEARCHED (Int. Cl.5)** |
| | | | C 12 P |

The present search report has been drawn up for all claims

| Place of search | Date of completion of the search | Examiner |
|---|---|---|
| THE HAGUE | 29-06-1990 | REMPP G.L.E. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document